# FASCICULE DE BREVET EUROPEEN

(11) **EP 2 179 926 B1**
(45) Date de publication et mention de la délivrance du brevet: **19.12.2012**
(21) Numéro de dépôt: 09173372.5
(22) Date de dépôt: 19.10.2009
(51) Int. Cl.: B65B 3/00, A61K 51/00, G01T 1/161

(54) **Dispositif stérile à usage unique de préparation d'un médicament radiopharmaceutique, système et procédé mettant en oeuvre ce dispositif**
Sterile Einwegvorrichtung zur Präparation eines radioaktiven Arzneimittels, System und Verfahren zur Anwendung dieser Vorrichtung
Sterile, single-use device for preparing a radiopharmaceutical drug, system and method implementing this device

(30) Priorité: 23.10.2008 FR 0857206
(43) Date de publication de la demande: 28.04.2010
(73) Titulaire: Commissariat à l'Énergie Atomique et aux Énergies Alternatives, 75015 Paris (FR)
(72) Inventeur: Vuillemard, Catherine, 91250 Saint Germain sur Corbeil (FR); Douysset, Guilhem, 78220 Viroflay (FR); Corpace, Olivier, 78000 Versailles (FR); Lotrus, Paul, 78000 Versailles (FR); Duboue, Bruno, 91300 Massy (FR); Coulon, Christine, 78530 Buc (FR)
(74) Mandataire: Ilgart, Jean-Christophe

(56) Documents cités:
- EP-A- 1 860 029
- WO-A-2005/002971
- WO-A-2007/042781
- US-A- 5 911 252
- US-A1- 2005 278 066
- ENSING G J: "Automated systems for the preparation of technetium -kits in hospitals" DEVELOPMENTS IN NUCLEAR MEDICINE, SPRINGER, DORDRECHT, NL, vol. 22, 1 janvier 1992 (1992-01-01), pages 49-54, XP008083184 ISSN: 0167-9074
- SOLANKI K: "The use of automation in radiopharmacy" HOSPITAL PHARMACIST, XX, GB, vol. 7, no. 4, 1 avril 2000 (2000-04-01), pages 94-98, XP002449955 ISSN: 1352-7967

## Description

### DOMAINE TECHNIQUE

L'invention concerne un dispositif stérile à usage unique de préparation d'un médicament radiopharmaceutique marqué par exemple avec des émetteurs de simples photons comme le technétium 99m [^{99m}Tc], et un système et un procédé de préparation d'un médicament radiopharmaceutique mettant en oeuvre ce dispositif.

### ÉTAT DE LA TECHNIQUE ANTÉRIEURE

Le domaine de l'invention est celui de la préparation de médicaments radiopharmaceutiques. De tels médicaments contenant des radioéléments artificiels appelés radionucléides sont employés à des fins diagnostiques ou thérapeutiques et utilisés dans les services de médecine nucléaire. Ces médicaments se présentent soit sous forme de spécialités pharmaceutiques contenant des radionucléides qui sont livrées prêtes à l'emploi, soit sous forme de préparations radiopharmaceutiques qui sont préparées *in situ* et extemporanément par marquage de molécules vectrices, désignées sous le vocable de « trousses » par l'homme du métier, avec un radionucléide choisi issu d'un générateur. Le radionucléide le plus fréquemment utilisé en médecine nucléaire est le technétium 99m (^{99m}Tc), qui est facilement disponible grâce au générateur ^{99m}Mo/^{99m}Tc et que l' on administre sous forme d'une solution de pertechnétate de sodium. Cette solution est obtenue par élution pour donner des éluats de technétium 99m sous forme de solutions stériles et apyrogènes. Plus précisément, les molécules vectrices formant ces trousses sont des substances stériles et apyrogènes, qui sont pré-conditionnées le plus souvent sous forme de flacons trousse fermés sous vide.

De manière connue, on utilise généralement pour la préparation de ces médicaments des enceintes blindées pourvues d'ouvertures de type ronds de gants sur les bords desquels sont fixés des gants en latex où les opérateurs introduisent les mains. Les préparations se font par transfert d'un éluat dilué dans le flacon trousse à l'aide de seringues munies de protèges-seringues plombés.

Les inconvénients majeurs de ces procédés de préparation traditionnels résident dans l'existence de débits de doses radioactives très élevés au contact des extrémités des doigts de l'opérateur lors de la manipulation des seringues et lors des mesures du flacon d'éluat de technétium 99m et du flacon trousse contenant l'éluat dilué. Les risques de contamination radioactive de l'opérateur lors de la préparation des médicaments sont significatifs.

Différents systèmes automatisés de préparation de médicaments radiopharmaceutiques permettent d'en faciliter la fabrication et surtout de résoudre le problème technique de contamination radioactive d'un opérateur en diminuant la dosimétrie des extrémités, ou « bout de doigt », des opérateurs, i.e. l'exposition des extrémités des doigts aux rayonnements radioactifs générés par les radionucléides, lors de la préparation de médicaments.

Le document référencé [1] en fin de description décrit un système automatisé de préparation de médicaments radiopharmaceutiques à visée industrielle. Les médicaments préparés sont destinés à être acheminés, par la suite, vers des services de médecine nucléaire. Ce système automatisé n'inclut pas de système de mesure de l'activité radioactive des solutions. Seuls les volumes de solution sont mesurés. L'activité radioactive est déduite à partir de la valeur de l'activité volumique mesurée en dehors du système automatisé. La mesure d'activité est effectuée, préalablement à la préparation des trousses, sur l'éluat, puis, une fois la trousse préparée, sur le produit final. Ces deux opérations de mesure peuvent conduire à l'exposition de l'opérateur aux rayonnements. D'autre part, dans le système décrit dans ce document, seul le prolongateur terminal raccordé au conteneur récipient (flacon contenant la molécule à marquer) ainsi que l'évent à gaz sont changés ou rincés après chaque préparation, ce qui n'est pas satisfaisant d'un point de vue pharmaceutique.

L'invention a pour objet de résoudre ces différents problèmes techniques en proposant un dispositif stérile, ou « kit », de préparation à usage unique, conditionné avant utilisation sous enveloppe fermée stérile, apte à être disposé dans un automate pouvant être introduit à l'intérieur d'une enceinte blindée et confinée prééxistante d'une installation de médecine nucléaire.

### EXPOSÉ DE L'INVENTION

L'invention concerne un dispositif stérile à usage unique de préparation d'un médicament radiopharmaceutique, apte à être inséré dans un automate, **caractérisé en ce qu**'il comprend une rampe munie :
- de cinq vannes à trois voies en série,
- d'un premier prolongateur raccordé, à sa première extrémité, à la première vanne et, à sa seconde extrémité, à une seringue tampon,
- d'un second prolongateur raccordé, à sa première extrémité, à la quatrième vanne, et, à sa seconde extrémité, à une seconde aiguille,
- d'un troisième prolongateur raccordé, à sa première extrémité, à la cinquième vanne, et, raccordé à sa seconde extrémité, à une troisième aiguille,
- d'un quatrième prolongateur muni d'une valve à clapet anti-retour, raccordé, à sa première extrémité, à une première extrémité de la rampe et, à sa seconde extrémité, à une quatrième aiguille apte à pénétrer dans un réservoir de dilution poche de chlorure de sodium,
- d'une première aiguille raccordée à la seconde vanne,
- d'un filtre à air relié à la troisième vanne,
- d'un bouchon fermant sa seconde extrémité.

Dans un exemple de réalisation avantageux, la rampe est réalisée dans un matériau transparent et compatible avec la radioactivité et les solutions employées. Cette rampe peut être en polysulfonate et/ou en polyéthylène. Le filtre à air peut être un filtre stérilisant. Les aiguilles sont par exemple des aiguilles de type BD Microlance (marque déposée) 3 (1,1 mm x 25 mm). Le réservoir de dilution peut contenir une solution de dilution isotonique injectable.

L'invention concerne également un système de préparation d'un médicament radiopharmaceutique comprenant un automate comportant deux chambres d'ionisation, disposé dans une enceinte blindée et confinée d'une installation de médecine nucléaire, relié à un système de mesure et de traitement disposé hors de cette enceinte, et le dispositif de l'invention intégré dans cet automate.

Avantageusement, ce système comprend :
- des moyens de commande électrique du piston de la seringue tampon,
- un réservoir contenant la solution de dilution suspendu en hauteur, placé à la première extrémité de la rampe,
- un flacon d'éluat pourvu de moyens de confinement des rayonnements ionisants, tel qu'une protection en tungstène et/ou en plomb, placés sur un support permettant de le maintenir retourné et positionné de manière à percuter la première aiguille,
- un flacon trousse contenant un composé vecteur à l'état lyophilisé pour l'obtention, par réaction chimique avec l'éluat dilué, dudit médicament ;
- un flacon déchet pourvu de moyens de confinement des rayonnements ionisants, tel qu'une protection en tungstène et/ou en plomb, destiné à recevoir la radioactivité résiduelle,
- des moyens de commande des cinq vannes.

Le système de l'invention permet de mesurer directement *in situ*, lors de la préparation du flacon trousse, l'activité radioactive du flacon d'éluat et de ce flacon trousse, sans avoir recours à l'intervention d'un opérateur. On limite ainsi l'irradiation « bout des doigts » et en même temps les risques d'accidents dus aux manipulations de substances radioactives. De plus, le dispositif de l'invention étant changé à chaque nouvelle fabrication de médicament, ceci garantit une parfaite stérilité et l'apyrogénécité des flacons trousse préparés.

L'invention concerne également un procédé de préparation in situ d'un médicament radiopharmaceutique, par exemple un médicament marqué avec des émetteurs de simples photos comme le technétium 99m, mettant en oeuvre ce système, comprenant les étapes suivantes :
(a) au moins une étape de sélection des paramètres et de positionnement du dispositif de l'invention sur l'automate ;
(b) une étape de mesure de l'activité de l'éluat dans la première chambre d'ionisation et de détermination de la quantité requise pour la préparation envisagée ;
(c) une étape de mélange de l'éluat avec la solution de dilution, adaptée au médicament radiopharmaceutique à produire ;
(d) une étape de reconstitution du flacon trousse et de mesure de l'activité radioactive dudit flacon trousse dans la seconde chambre d'ionisation ;
(e) une étape de nettoyage du dispositif de l'invention, les résidus étant envoyés vers le flacon déchet ;
(f) une étape de retrait du flacon trousse contenant le médicament radiopharmaceutique préparé, en vue de son utilisation.

Le procédé de l'invention est donc entièrement automatisé depuis le positionnement du dispositif de l'invention dans l'automate jusqu'à l'obtention finale du médicament radiopharmaceutique de telle manière que les mains d'un opérateur ne soient à aucun moment exposées aux rayonnements radioactifs générés par le radionucléide, ce qui permet de diminuer la dosimétrie « bout de doigt » des préparateurs et des manipulateurs en électroradiologie, ainsi que les risques d'accident.

### BRÈVE DESCRIPTION DES DESSINS

La figure 1 est une vue schématique du dispositif de l'invention.
La figure 2 est une vue schématique générale d'un automate dans lequel peut être intégré le dispositif de l'invention.
Les figures 3 à 8 sont des vues schématiques illustrant des étapes du procédé de l'invention.

### EXPOSÉ DÉTAILLÉ DE MODES DE RÉALISATION PARTICULIERS

L'invention concerne un dispositif stérile à usage unique, ou « kit », de préparation d'un médicament radiopharmaceutique permettant le transfert de produits radioactifs, apte à être positionné au sein d'un automate.

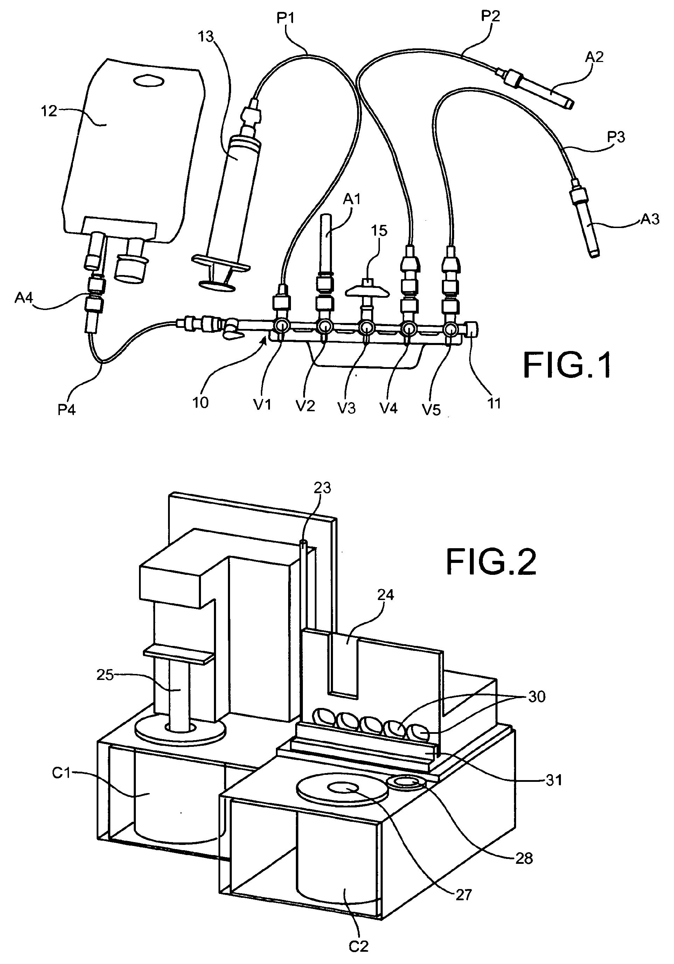

Comme illustré sur la figure 1, ce dispositif comporte une rampe 10 pourvue :
- de cinq vannes à trois voies V1 à V5 en série,
- à partir de la première vanne V1, d'un premier prolongateur P1 raccordé à une seringue 13 dite « seringue tampon », et d'un quatrième prologation P4 muni d'une valve à clapet anti-retour, raccordé en une première extrémité de la rampe 10, à une quatrième aiguille A4 apte à pénétrer dans un réservoir de dilution poche de chlorure de sodium 12,
- d'une première aiguille A1 au niveau de la seconde vanne V2,
- d'un filtre à air stérilisant 15 relié à la troisième vanne V3,
- d'un second prolongateur P2 à partir de la quatrième vanne V4, raccordé à une seconde aiguille A2,
- d'un troisième prolongateur P3 à partir de la cinquième vanne V5, raccordé à une troisième aiguille A3,
- d'un bouchon 11 à une seconde extrémité de la rampe 10.

La première aiguille A1 permet de percuter un flacon d'éluat 20 afin de permettre le transfert de l'éluat E dans la seringue 13.

La seconde aiguille A2 permet de percuter le flacon trousse 21 afin de permettre le transfert de l'éluat dilué dans le flacon trousse 21.

La troisième aiguille A3 permet de percuter le flacon de déchets 22 afin de permettre le transfert des déchets dans le flacon de déchets 22.

La quatrième aiguille A4 permet de percuter le réservoir de dilution poche de chlorure de sodium 12 pour transférer de la solution dans la seringue 13.

Dans un exemple de réalisation avantageux, la rampe 10 est réalisée dans un matériau transparent et compatible avec la radioactivité et les solutions employées. De préférence cette rampe est en polysulfonate et/ou en polyéthylène. Les prolongateurs P1, P2, P3 et P4 sont réalisés par exemple en silicone, de diamètre interne 1 mm et de longueur environ 30 cm. Le prolongateur P4 peut toutefois avoir une longueur d'environ 15 cm. Le filtre à air stérilisant a avantageusement des pores de diamètres 0,22 µm. Les aiguilles A1, A2, A3 et A4 sont par exemple des aiguilles de type BD microlance 3 (1, 1 mm x 25 mm). Le bouchon peut être un bouchon qui se visse et se verrouille. Le réservoir de dilution 12 contient une solution de dilution isotonique injectable, par exemple une dilution de chlorure de sodium 0,9%.

Dans un système selon l'invention de mise en oeuvre du dispositif de l'invention illustré sur la figure 1, celui-ci est intégré dans un automate, illustré sur la figure 2, destiné à la préparation in situ de médicaments radiopharmaceutiques, introduit à l'intérieur d'une enceinte blindée et confinée préexistante d'une installation de médecine nucléaire. Cet automate est pourvu de deux moyens de mesure de l'activité radioactive, ce qui permet la mesure automatique sans intervention humaine de l'activité de l'éluat dans la seringue 13 et de l'éluat dilué dans le flacon trousse 21. Cet automate est de plus relié à un système informatique 26 positionné hors de l'enceinte blindée pour la commande automatisée de ses différents organes. La production du flacon trousse 21 est ainsi entièrement automatisée après le positionnement du dispositif de l'invention dans l'automate. Ces moyens de mesure, calibrés pour le radionucléide et le conditionnement envisagés, permettent de déterminer l'activité de l'éluat et l'activité du flacon trousse préparé. De façon avantageuse, ces moyens de mesure sont des chambres d'ionisation C1 et C2 adaptées au radionucléide à détecter, entourées d'un blindage, par exemple métallique, permettant de les isoler de l'ambiance radioactive environnante.

En effet, un volume d'éluat issu d'un générateur de radionucléide, par exemple du technétium, est dilué dans une solution avant d'être mis en contact avec un composé vecteur pour constituer un flacon trousse 21, tout en permettant :
- la mesure de l'activité radioactive de l'éluat, par exemple du pertechnétate de sodium,
- la mesure de l'activité radioactive du flacon trousse préparé.

La première chambre d'ionisation C1 est munie d'un support permettant de maintenir la seringue tampon 13 en position de mesure. De la même façon, la seconde chambre d'ionisation C2 est munie d'un support permettant de maintenir le flacon trousse 21 en position de mesure.

Les deux chambres d'ionisation C1 et C2 sont connectées à au moins un système de mesure du signal électrique délivré, doté d'une interface de communication avec le système informatique 26 pilotant l'automate.

Les deux chambres d'ionisation C1 et C2 peuvent être pressurisées ou non pressurisées. Dans le cas où elles ne sont pas pressurisées, il est nécessaire de leur adjoindre, en proximité immédiate, un système de mesure des paramètres atmosphériques tels que la température, la pression et l'humidité relative pour corriger le signal délivré des variations de la densité de l'air ambiant.

Comme illustré sur la figure 2, l'automate comporte, en outre, un moyen de positionnement et de fixation de la rampe 10 constituant le dispositif de l'invention. Ce positionnement est préférentiellement opéré par glissement de ladite rampe sur un support adapté 31. La rampe 10 une fois placée sur son support est ainsi bloquée en position fixe, les orifices 30 correspondant au passage des vannes V1 à V5.

En outre, le système de l'invention comprend notamment :
- des moyens de commande électrique du piston de la seringue tampon 13, placé, par exemple de manière verticale, en regard de la première vanne V1, constitué d'un pousse seringue électrique 25 ou d'un système analogue tel qu'une pompe électrique, et destiné à commander le remplissage et l'extraction de ladite seringue ;
- un réservoir 12 contenant la solution de dilution, suspendu en hauteur, via un support métallique 23 approprié placé à la première extrémité de la rampe 10, constitué d'une poche souple contenant une solution de dilution isotonique injectable, par exemple une dilution physiologique de NaCl à 0,9% pour permettre la dilution de l'éluat E ;
- un flacon d'éluat 20 pourvu d'un moyen de confinement des rayonnements ionisants, tel qu'une protection en tungstène et/ou en plomb, placé sur un support 24 permettant de le maintenir retourné et positionné de manière à percuter l'aiguille placée au niveau de la seconde vanne V2 de façon à permettre le transfert de l' éluat E dans la seringue tampon 13 sous la commande de ces moyens de commande électrique du piston ;
- un flacon trousse 21 contenant un composé vecteur à l'état lyophilisé pour l'obtention, par réaction chimique avec l'éluat E dilué, du médicament souhaité ;
- un flacon déchet 22 pourvu d'un moyen de confinement des rayonnements ionisants, tel qu'une protection en tungstène et/ou en plomb, destiné à recevoir la radioactivité résiduelle éventuellement stockée au niveau du dispositif de préparation de l'invention,
- des moyens de commande des cinq vannes du dispositif de l'invention.

Cet automate présente une grande compacité qui le rend adapté à l'ensemble des enceintes confinées et blindées utilisées en médecine nucléaire, et notamment en tomographie d'émission monophotonique (« TEMP » en abrégé). L'encombrement de la partie de l'automate placée dans l'enceinte blindée est inférieur à 0,15 m². Cet automate peut être ainsi introduit à l'intérieur d'une enceinte blindée et confinée préexistante d'une installation de médecine nucléaire, par au moins une porte de l'enceinte délimitant une ouverture par exemple de superficie inférieure à 0,2 m² et, de préférence, inférieure à 0,1 m².

La présente invention concerne également un procédé de préparation *in situ* de médicaments radiopharmaceutiques, mettant en oeuvre le système décrit ci-dessus. Ce procédé comprend notamment les étapes suivantes :
a) au moins une étape de sélection des paramètres et de positionnement du dispositif de l'invention sur l'automate ;
b) une étape de mesure de l'activité de l'éluat E dans la première chambre d'ionisation C1 et de détermination de la quantité requise pour la préparation envisagée ;
c) une étape de mélange de l'éluat avec la solution de dilution, adaptée au médicament radiopharmaceutique à produire ;
d) une étape de reconstitution du flacon trousse 21 et de mesure de l'activité radioactive dudit flacon trousse 21 dans la seconde chambre d'ionisation C2 ;
e) une étape de nettoyage du dispositif de l'invention, les résidus étant envoyés vers le flacon déchet 22 ;
f) une étape de retrait du flacon trousse 21 contenant le médicament radiopharmaceutique préparé, en vue de son utilisation.

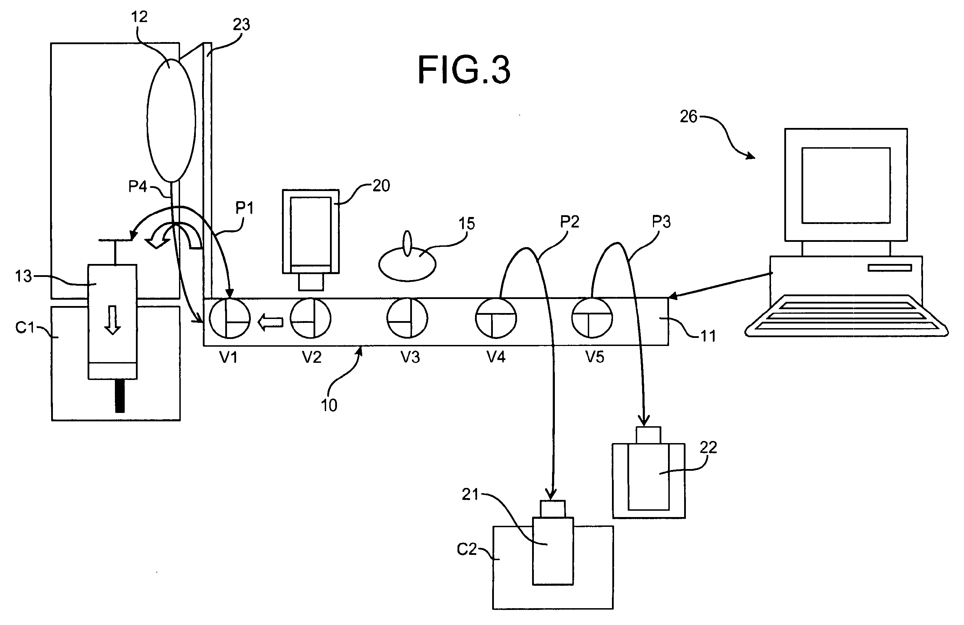

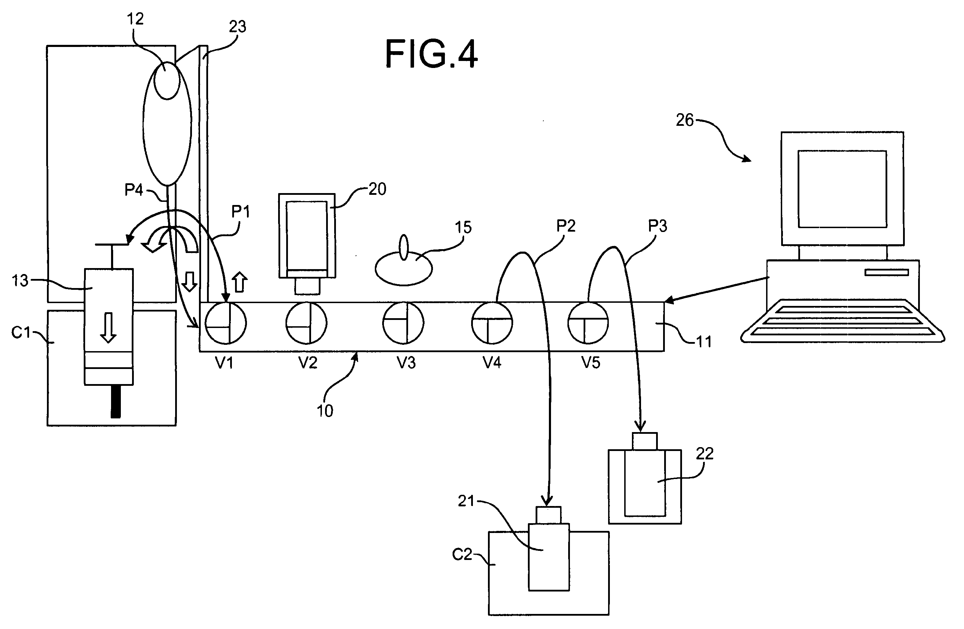

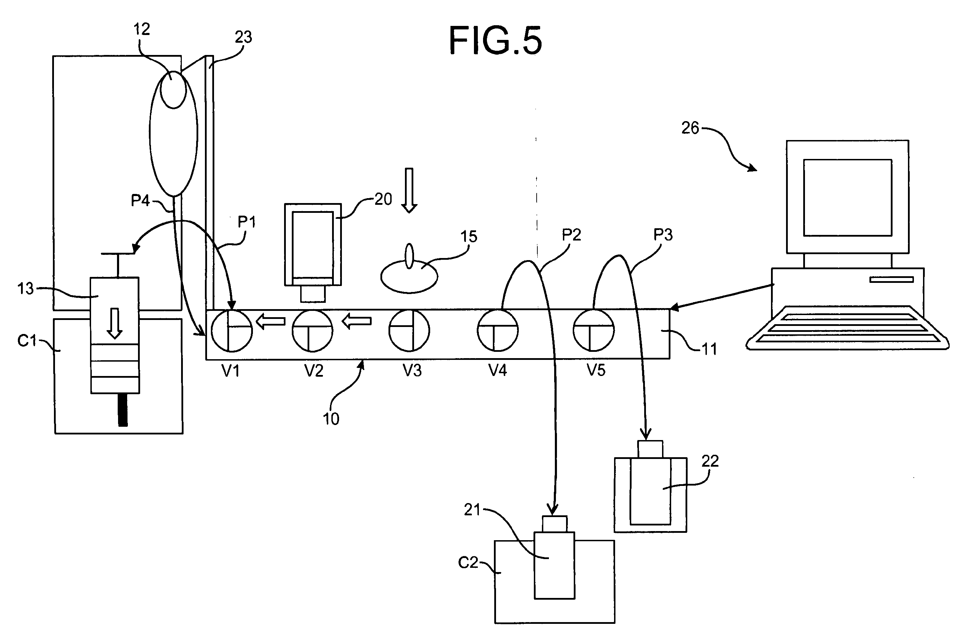

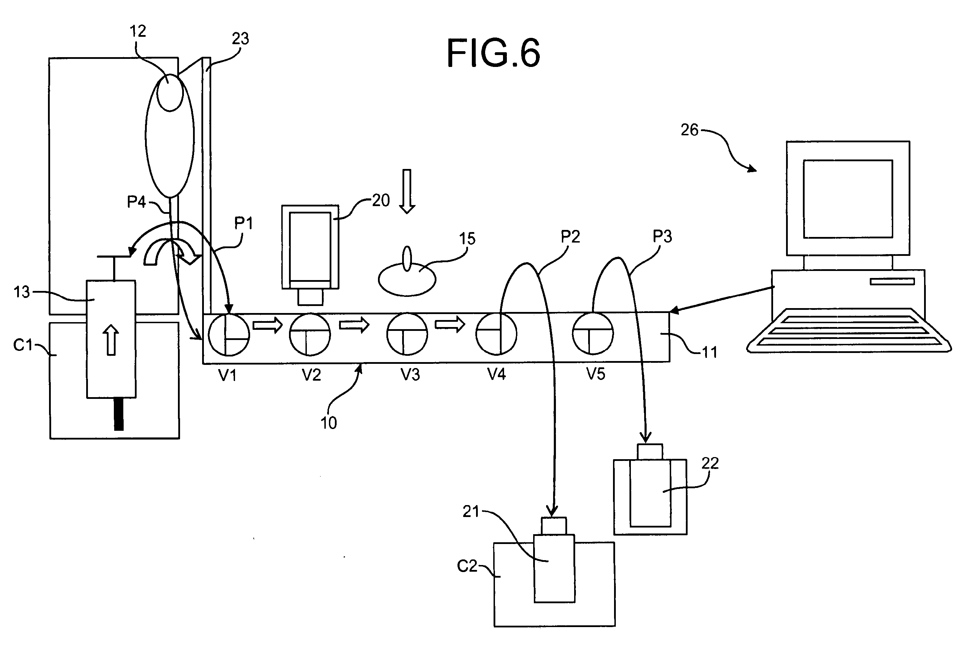

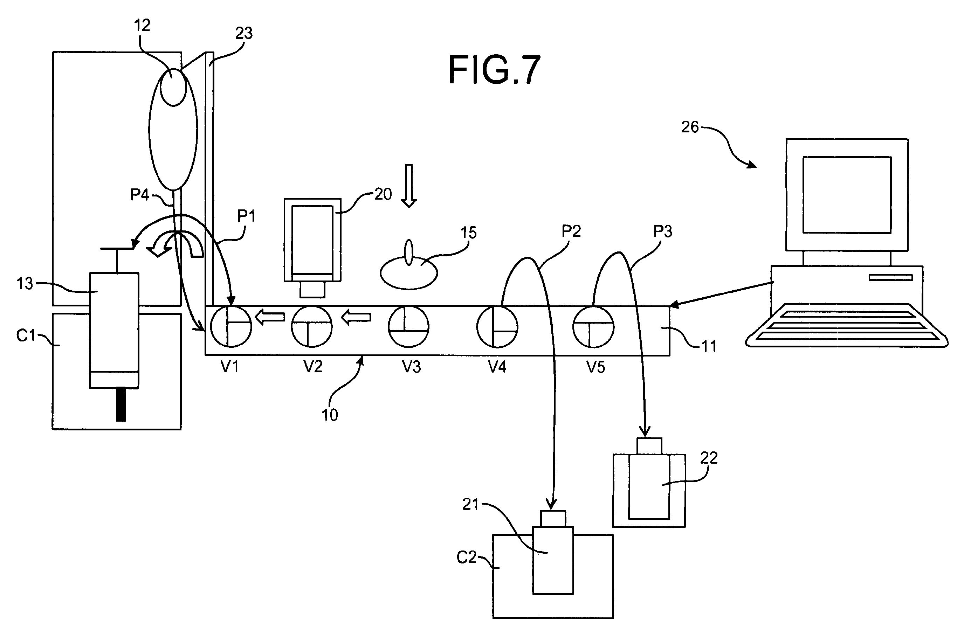

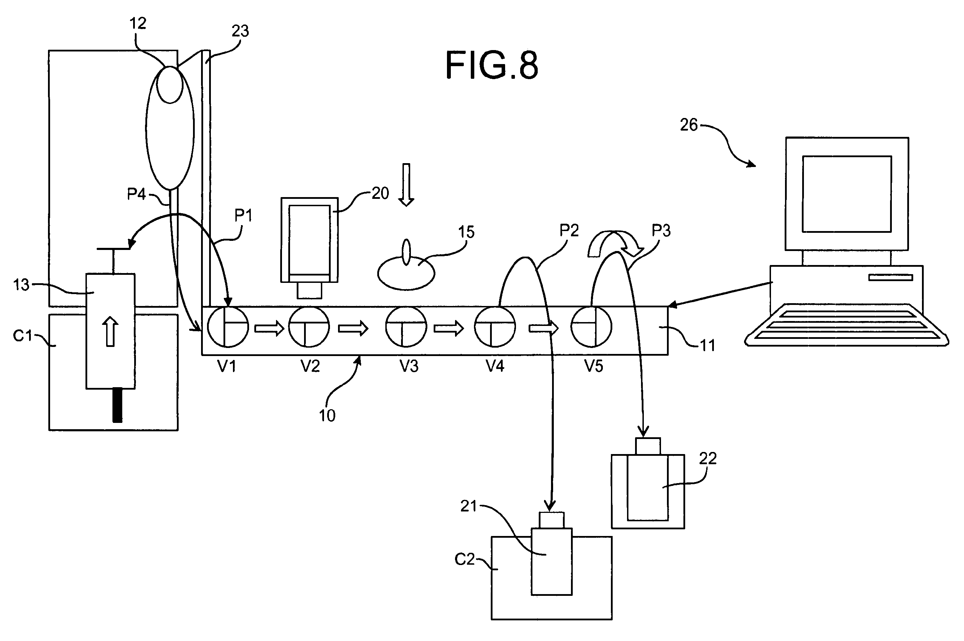

Analysons plus précisément le contenu de chacune de ces étapes :
a) Lors de la première étape, on choisit, dans le logiciel de préparation implanté dans le système informatique 26, le programme du médicament radiopharmaceutique à préparer et on saisit l'activité et le volume à incorporer dans le flacon trousse 21. Le volume de l'éluat E peut être déterminé par pesée différentielle à l'aide d'une balance qualifiée avant et après élution. Le dispositif de l'invention est alors positionné sur l'automate, en suivant les indications données par le logiciel :
   - La rampe 10 est introduite par glissement sur le support prévu 31.
   - La seringue tampon 13 est introduite dans la première chambre d'ionisation C1 sur son support et positionnée ensuite sur le pousse seringue 25.
   - Le réservoir de dilution 12 est fixé en 23 au-dessus de la rampe 10 et communique avec elle par le biais du quatrième prolongateur P4.
   - Le flacon trousse 21 contenant la molécule à marquer est positionné en 27 dans la deuxième chambre d'ionisation C2 sur son support et est raccordé à la rampe 10 par l'intermédiaire du second prolongateur P2 muni de son aiguille.
   - Le flacon déchets 22 est positionné à l'emplacement prévu 28 dans sa protection en tungstène et/ou en plomb et est raccordé à la rampe 10 par l'intermédiaire du troisième prolongateur P3 muni de son aiguille A3.
   Après élution et pesée du flacon d'éluat 20 muni de sa protection en tungstène et/ou en plomb, celui-ci est positionné par retournement sur l'automate au niveau de la seconde vanne V2 percutant ainsi la première aiguille A1 maintenue par un moyen de raccordement au niveau de la rampe 10.
   On lance la préparation en validant le positionnement effectif du dispositif de préparation de l'invention.
b) La seconde étape consiste à prélever à l'aide de la seringue tampon 13 la totalité du volume de l'éluat E contenu dans le flacon d'éluat 20 pour en mesurer l'activité radioactive totale grâce à la première chambre d'ionisation C1. L'activité et le volume sont enregistrés par le logiciel de pilotage et la totalité de l'éluat prélevé est ensuite replacé dans le flacon d'éluat 20, par extraction de la seringue tampon 13. Comme illustré sur la figure 3, la première vanne V1 est positionnée ouverte à droite, la seconde vanne V2 est positionnée ouverte à gauche et la troisième vanne V3 ouverte à gauche.
   La seringue tampon 13 prélève ensuite la quantité d'éluat nécessaire en fonction de l'activité requise pour la préparation envisagée. Le remplissage de ladite seringue 13 est contrôlé en continu par le signal délivré par la première chambre d'ionisation C1. Le prélèvement est arrêté lorsque l'activité demandée est contenue dans la seringue tampon 13.
c) La troisième étape consiste à diluer l'éluat prélevé par la seringue tampon 13 au cours de la seconde étape avec une solution de NaCl à 0,9% en provenance du réservoir de dilution 12 afin d'obtenir la dilution nécessaire au flacon trousse 21 en fonction du médicament radiopharmaceutique à préparer. Pour cela, comme illustré sur la figure 4, la première vanne V1 est actionnée avec ouverture à gauche et la seringue tampon 13 aspire le volume de chlorure de sodium 0,9% nécessaire à la réalisation du médicament radiopharmaceutique. A la fin de la dilution, comme illustré sur la figure 5, la première vanne V1 est positionnée ouverte à droite, la seconde vanne V2 ouverte vers le bas et la troisième vanne V3 ouverte à gauche de façon à aspirer le volume restant au niveau de la rampe 10 ainsi que de l'air filtré.
d) La quatrième étape est l'étape de reconstitution du flacon trousse 21. L'éluat dilué obtenu lors de la troisième étape est alors transféré vers le flacon trousse 21 contenant le composé vecteur à l'état lyophilisé, ledit flacon trousse 21 étant positionné sur un support adapté à l'intérieur de la seconde chambre d'ionisation C2. Comme illustré sur la figure 6, la première vanne V1 est actionnée ouverte à droite, les seconde et troisième vannes V2 et V3 sont positionnées ouvertes vers le bas et la quatrième vanne V4 est actionnée ouverte à gauche. La mesure calibrée de l'activité radioactive du flacon trousse 21 est alors effectuée et la valeur obtenue est transférée au système informatique 26 de contrôle de l'automate.
   La programmation informatique initiale doit tenir compte du volume résiduel et, par conséquent, de l'activité résiduelle restant dans le dispositif de l'invention après transfert dans le flacon trousse 21.
e) La cinquième étape est une étape de nettoyage, réalisée par actionnement ouverture à droite de la première vanne V1, la seconde vanne V2 étant positionnée ouverte vers le bas, la troisième vanne V3 ouverte vers le haut, la quatrième vanne V4 ouverte à droite et la cinquième vanne V5 ouverte vers le bas, comme illustré sur la figure 7. La seringue tampon 13 prélève un volume d'air filtré suffisant par le filtre 15 et par orientation ouverture vers le bas des seconde, troisième et quatrième vannes V2, V3 et V4 et en position ouverture à gauche de la cinquième vanne V5, comme illustré sur la figure 8, et dirige les résidus vers un flacon de déchet 22 connecté au troisième prolongateur P3.
   La préparation du médicament radiopharmaceutique est terminée. Les dates de fabrication et de mesure de l'activité radioactive de l'éluat et de chaque flacon trousse 21 sont enregistrées par le système informatique 26.
f) La sixième étape consiste à retirer le flacon trousse 21 de la seconde chambre d'ionisation, en vue de son utilisation.

## Revendications

1. Dispositif stérile de préparation d'un médicament radiopharmaceutique à usage unique, apte à être inséré dans un automate, **caractérisé en ce qu'**il comprend une rampe (10) munie :
- de cinq vannes à trois voies en série (V1, V2, V3, V4, V5),
- d'un premier prolongateur (P1) raccordé, à sa première extrémité, à la première vanne (V1) et, à sa seconde extrémité, à une seringue tampon (13),
- d'un second prolongateur (P2) raccordé, à sa première extrémité, à la quatrième vanne (V4), et, à sa seconde extrémité, à une seconde aiguille (A2),
- d'un troisième prolongateur (P3) raccordé, à sa première extrémité, à la cinquième vanne (V5), et, à sa seconde extrémité, à une troisième aiguille (A3),
- d'un quatrième prolongateur (P4) muni d'une valve à clapet anti-retour, raccordé, à sa première extrémité, à une première extrémité de la rampe (10) et, à sa seconde extrémité, à une quatrième aiguille (A4) apte à pénétrer dans un réservoir de dilution poche de chlorure de sodium (12),
- d'une première aiguille (A1) raccordée à la seconde vanne (V2),
- d'un filtre à air (15) relié à la troisième vanne (V3),
- d'un bouchon (11) fermant sa seconde extrémité.

2. Dispositif selon la revendication 1, dans lequel la rampe (10) est en polysulfonate et/ou polyéthylène.

3. Dispositif selon la revendication 1, dans lequel les prolongateurs (P1, P2, P3, P4) sont réalisés en silicone.

4. Dispositif selon la revendication 1, dans lequel le filtre à air (15) est un filtre stérilisant.

5. Dispositif selon la revendication 1, dans lequel le réservoir de dilution contient une solution de dilution isotonique injectable.

6. Système de préparation d'un médicament radiopharmaceutique comprenant un automate comportant deux chambres d'ionisation, disposé dans une enceinte blindée et confinée d'une installation de médecine nucléaire, relié à un système de mesure et de traitement disposé hors de cette enceinte, et le dispositif selon l'une quelconque des revendications précédentes intégré dans cet automate.

7. Système selon la revendication 6, qui comprend :
- des moyens de commande électrique du piston de la seringue tampon (13),
- un réservoir (12) contenant la solution de dilution suspendu en hauteur, placé à la première extrémité de la rampe (10),
- un flacon d'éluat (20) pourvu de moyens de confinement des rayonnements ionisants, placés sur un support permettant de les maintenir retourné et positionné de manière à percuter la première aiguille,
- un flacon trousse (21) contenant un composé vecteur à l'état lyophilisé pour l'obtention, par réaction chimique avec l'éluat (E) dilué, dudit médicament ;
- un flacon déchet (22) pourvu d'un moyen de confinement des rayonnements ionisants, destiné à recevoir la radioactivité résiduelle,
- des moyens de commande des cinq vannes (V1-V5).

8. Procédé de préparation in situ d'un médicament radiopharmaceutique mettant en oeuvre le système selon l'une quelconque des revendications 6 ou 7, comprenant les étapes suivantes :
a) au moins une étape de sélection des paramètres et de positionnement du dispositif sur l'automate ;
b) une étape de mesure de l'activité de l'éluat (E) dans la première chambre d'ionisation (C1) et de détermination de la quantité requise pour la préparation envisagée ;
c) une étape de mélange de l'éluat (E) avec la solution de dilution, adaptée au médicament radiopharmaceutique à produire ;
d) une étape de reconstitution du flacon trousse (21) et de mesure de l'activité radioactive dudit flacon trousse (21) dans la seconde chambre d'ionisation (C2) ;
e) une étape de nettoyage du dispositif, les résidus étant envoyés vers le flacon déchet (22) ;
f) une étape de retrait du flacon trousse (21) contenant le médicament radiopharmaceutique préparé.

9. Procédé selon la revendication 8, dans lequel le médicament radiopharmaceutique est marqué avec des émetteurs de simples photons comme le technétium 99m.

## Claims

1. A sterile device for preparing a single-use radiopharmaceutical, able to be inserted into an automatic machine, **characterised in that** it includes a manifold (10) fitted:
- with five three-way valves in series (V1, V2, V3, V4, V5),
- with a first connecting tube (P1) connected, at its first end, to the first valve (V1) and, at its second end, to a buffer syringe (13),
- with a second connecting tube (P2) connected, at its first end, to the fourth valve (V4) and, at its second end, to a second needle (A2),
- with a third connecting tube (P3) connected, at its first end, to the fifth valve (V5) and, at its second end, to a third needle (A3),
- with a fourth connecting tube (P4) fitted with a nonreturn valve connected, at its first end, to a first end of the manifold (10) and, at its second end, to a fourth needle (A4) able to penetrate into a diluent bag of sodium chloride (12),
- with a first needle (A1) connected to the second valve (V2),
- with an air filter (15) connected to the third valve (V3),
- with a stopper (11) sealing its second end.

2. A device according to claim 1, in which the manitoid (10) is made of polysulfonate and/or polyethylene.

3. A device according to claim 1, in which the connecting tubes (P1, P2, P3, P4) are made of silicon.

4. A device according to claim 1, in which the air filter (15) is a sterilising filter.

5. A device according to claim 1, in which the diluent bag contains an injectable isotonic diluent solution.

6. A system for preparing a radiopharmaceutical including an automatic machine having two ionisation chambers, where the automatic machine is positioned in a shielded and confined enclosure of a nuclear medicine installation, connected to a measurement and processing system positioned outside this enclosure, and the device according to any of the previous claims incorporated in this automatic machine.

7. A system according to claim 6, which includes:
- means of electrical control of the piston of the buffer syringe (13),
- a bag (12) containing the diluent solution suspended at height, installed at the first end of the manifold (10),
- a flask of eluate (20) having means of confinement of ionising radiation, installed on a bracket able to hold them, which is turned and positioned so as to strike the first needle,
- a bag flask (21) containing a carrier compound in a freeze-dried state for obtaining the said radiopharmaceutical, by means of a chemical reaction with the diluted eluate (E);
- a waste flask (22) having a means of confinement of the ionising radiation, intended to receive the residual radioactivity,
- means for controlling the five valves (V1-V5).

8. A method for in situ preparation of a radiopharmaceutical using the system according to either of claims 6 or 7, including the following steps:
a) at least one step of selection of the parameters and positioning of the device on the controller;
b) a step of measurement of the activity of the eluate (E) in the first ionisation chamber (C1), and of determination of the quantity required for the envisaged preparation;
c) a step of blending of the eluate (E) with the diluent solution, suitable for the radiopharmaceutical to be produced;
d) a step of reconstitution of the bag flask (21) and of measurement of the radioactive activity of the said bag flask (21) in the second ionisation chamber (C2);
e) a step of cleaning of the device, where the residues are sent to the waste flask (22);
f) a step of removal of the bag flask (21) containing the prepared radiopharmaceutical.

9. A method according to claim 8, in which the radiopharmaceutical is marked with emitters of single photons such as technetium 99m.

## Patentansprüche

1. Sterine Vorrichtung zur Herstellung eines radio -pharmazeutischen Medikaments für einmaligen Gebrauch, das in einen Automaten eingeführt werden kann, **dadurch gekennzeichnet, dass** sie eine Rampe bzw. Leiste (10) umfasst, welche mit Folgendem versehen ist:
-- fünf Drei-Wege-Ventilen (V1, V2, V3, V4, V5) in Reihe,
-- einer ersten Verlängerungsleitung (P1), die an ihrem ersten Ende mit dem ersten Ventil (V1) und an ihrem zweiten Ende mit einer Puffer- bzw. Tamponspritze (13) verbunden ist,
-- einer zweiten Verlängerungsleitung (P2), die an ihrem ersten Ende mit dem vierten Ventil (V4) und an ihrem zweiten Ende mit einer zweiten Einspritznadel bzw. Injektionsspritze (A2) verbunden ist,
-- einer dritten Verlängerungsleitung (P3), die an ihrem ersten Ende mit dem fünften Ventil (V5) und an ihrem zweiten Ende mit einer dritten Einspritznadel bzw. Injektionsspritze (A3) verbunden ist,
-- einer mit einer Rückschlagventilklappe versehenen vierten Verlängerungsleitung (P4), die an ihrem ersten Ende mit einem ersten Ende der Rampenleiste (10) und an ihrem zweiten Ende mit einer vierten Einspritznadel bzw. lnjektionsspritze (A4) verbunden ist, die in einen Verdünnungs-Behälterbeutel (12) mit Natriumchlorid einführbar ist ,
-- einer mit dem zweiten Ventil (V2) verbundenen ersten Einspritznadel bzw. Injektionsspritze (A1),
-- einem mit dem dritten Ventil (V3) verbundenen Luftfilter (15)
-- einem ihr zweites Ende verschließenden Verschlußstopfen (11).

2. Vorrichtung nach Anspruch 1, bei welcher die Rampe bzw. Leiste (10)aus Polysulfonat und/oder aus Polyäthylen besteht.

3. Vorrichtung nach Anspruch 1, bei welcher die Verlängerungsleitungen (P1, P2, P3, P4) aus Silikon hergestellt sind.

4. Vorrichtung nach Anspruch 1, bei welcher das Luftfilter (15) ein Sterilisierfilter ist.

5. Vorrichtung nach Anspruch 1, bei welcher der Verdünnungsmittelbehälter ein injektionsfähiges isotonisches Verdünnungsmittel enthält.

6. System zur Herstellung eines radiopharmazeutischen Medikaments, das System umfassend einen zwei Ionisationskammern enthaltenden Automaten, der in einer abgeschirmten und abgegrenzten Einschließung einer nuklearmedizinischen Anlage angeordnet und mit einem außerhalb dieser Einschließung angeordneten Mess- und Behandlungssystem verbunden ist, sowie weiter umfassend die Vorrichtung nach einem beliebigen der vorhergehenden Ansprüche, integriert in diesen Automaten.

7. System nach Anspruch 6, umfassend:
-- Mittel zur elektrischen Steuerung des Kolbens der Puffer- bzw. Tampon-Spritze (13),
-- einen erhöht aufgehängten Vorratsbehälter (12) mit der Verdtinnungslösung, in Anordnung am ersten Ende der Rampenleiste (10),
-- einen Eluat- bzw. Auswaschflakon (20), der mit Mitteln zur Eingrenzung ionisierender Strahlungen versehen ist, in Anordnung auf einer Halterung, welche die Eingrenzung dieser Strahlungen gewährleistet, und in Positionierung zum Durchstich der ersten Einspritznadel bzw. Injektionsspritze,
-- einen eine Vektor- bzw. Trägerverbindung enthaltenden Träger- bzw. Vektorflakon, für die Gewinnung des Medikaments durch chemische Reaktion mit dem verdünnten Eluat;
-- einen mit einem Mittel zur Einschließung ionisierender Strahlungen versehehenen Abfall-Flakon (22), zur Aufnahme der residuellen Radioaktivität,
-- Mittel zur Steuerung der fünf Ventile (V1 - V5).

8. Verfahren zur In-situ-Herstellung eines radiopharmazeutischen Medikaments, unter Verwendung des Systems gemäß einem beliebigen der Anspruch 6 oder 7, das Verfahren umfassend die folgenden Verfahrensschritte bzw. -stufen:
-- wenigstens eine Stufe der Auswahl von Parametern und der Positionierung der Vorrichtung in bzw. auf dem Automaten,
-- eine Stufe der Messung der Aküvität des Eluats (E) in der ersten Ionisationskammer (C1), und der Bestimmung der für die in Aussicht genommene Herstellung erforderlichen Menge,
-- eine Stufe der Mischung des Eluates (E) mit der Verdünnungslösung, in Anpassung an das herzustellende radiopharmazeutische Medikament,
-- eine Stufe der Rekonsütution des Träger- bzw. Vektorflakons (21) und zur Messung der Radioaktivität dieses Träger- bzw- Vektorflakons (21) in der zweiten Ionisationskammer (22).
-- eine Stufe der Reinigung der Vorrichtung, wobei die Rückstände dem Abfall-Flakon (22) zugeleitet werden,
-- eine Stufe des Einholens bzw. der Aufbereitung des Träger- bzw. Vektorflakons (21), welcher das hergestellte radiopharmazeutische Medikament enthält.

9. Verfahren nach Anspruch 8, bei welchem das radiopharmazeutische Medikament mit Emittern einfacher Photonen wie beispielsweise Technetium 99m maskiert wird.
